(19) Europäisches Patentamt
European Patent Office
Office européen des brevets

(11) Veröffentlichungsnummer : **0 058 924**
**B1**

(12) **EUROPÄISCHE PATENTSCHRIFT**

(45) Veröffentlichungstag der Patentschrift : **02.01.86**

(51) Int. Cl.[4] : **C 07 C101/48**, C 07 C103/84, C 07 D235/26, C 07 D241/52

(21) Anmeldenummer : **82101146.7**

(22) Anmeldetag : **17.02.82**

# (54) Verfahren zur Herstellung aromatischer Aminocarbonsäurealkylester.

(30) Priorität : **19.02.81 DE 3106111**

(43) Veröffentlichungstag der Anmeldung : **01.09.82 Patentblatt 82/35**

(45) Bekanntmachung des Hinweises auf die Patenterteilung : **02.01.86 Patentblatt 86/01**

(84) Benannte Vertragsstaaten : **CH DE FR GB LI**

(56) Entgegenhaltungen :
**US-A- 3 218 349**
**CHEMICAL ABSTRACTS, Band 82, Nr. 25, 23. Juni 1975, Seite 482, Nr. 170288k, Columbus, Ohio, USA, T. KIERSZNICKI et al.: "O-Alkylation of carboxylic acid salts with alkyl sulfates in dipolar aprotic solvents"**
**Beilsteins Handbuch der Organischen Chemie, 4. Auflage, III. Ergänzungswerk, 14. Band, 2. Teil, Seite 954**

(73) Patentinhaber : **HOECHST AKTIENGESELLSCHAFT**
**Postfach 80 03 20**
**D-6230 Frankfurt am Main 80 (DE)**

(72) Erfinder : **Hohn, Jürgen, Dr.**
**Egerländer Strasse 26**
**D-6233 Kelkheim (Taunus) (DE)**
Erfinder : **Hunger, Klaus, Dr.**
**Johann-Strauss-Strasse 35**
**D-6233 Kelkheim (Taunus) (DE)**

**EP 0 058 924 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Veresterung aromatischer Aminocarbonsäuren.

Aus Beilstein, E III, Bd. 14/2, Seite 954, ist bekannt, daß 4-Chlor-2-aminobenzoesäure mit Dimethylsulfat in wäßrig alkalischem Milieu zur 4-Chlor-2-methylaminobenzoesäure N-methyliert wird.

Aus Chem. Abstr. *82*, Seite 482 (1975), C.A. Nr. 170288 k, ist bekannt, Natriumsalze von o- oder p-Nitro- oder o-Hydroxybenzoesäure mit Dimethylsulfat in Dimethylsulfoxid bei 20 °C in einer Ausbeute von 91-98 % zu den entsprechenden Benzoesäuremethylestern umsetzen zu können. Die Veresterung einer aromatischen Aminocarbonsäure wird dabei nicht erwähnt.

Weiterhin ist aus der US-A-3,218,349 bekannt, Tetraiodanthranilsäure-Natriumsalz mit Dialkylsulften in Gegenwart von Kaliumcarbonat im Dimethylmethylformamid zu den entsprechenden Alkylestern zu verestern. Das Dialkylsulfat wird bei diesem Verfahren in einer Portion zugegeben und bei Raumtemperatur umgesetzt. Die üblich sauer katalysierten Veresterungen versagen bei dieser speziellen Aminocarbonsäure, die eine sterisch gehinderte Carboxylgruppe und eine sterisch gehinderte Aminogruppe besitzt. Eine Übertragbarkeit der erzielten hohen Selektivität und Ausbeute auf andere aromatische Aminocarbonsäuren ist deshalb nicht zu erwarten.

Es ist bereits aus M. *99* (1968) 102, 110 bekannt daß p-Aminobenzoesäure-methylester durch Umsetzung von p-Aminobenzoesäure mit Dimethylsufat in Dimethylformamid in Gegenwart von Kaliumcarbonat in 70 %iger Ausbeute erhalten werden kann. Die Ausgangsverbindungen werden bei diesem Verfahren bei 60 °C 15 Minuten unter Rühren miteinander umgesetzt, ohne dabei das entstehende Reaktionswasser aus der Reaktionsmischung zu entfernen oder zu binden.

Überraschenderweise wurde nun gefunden, daß Aminocarbonsäurealkylester in bedeutend höherer Ausbeute und mit ausgezeichneter Selektivität erhalten werden, wenn man statt der freien Aminocarbonsäuren deren Salze in einem wasserfreien Medium umsetzt. Die N-Alkylderivate entstehen hierbei höchstens spurenweise.

Gegenstand der Erfindung ist ein Verfahren zur Herstellung von niederen aromatischen Aminocarbonsäurealkylestern von Aminocarbonsäuren der allgemeinen Formel (I)

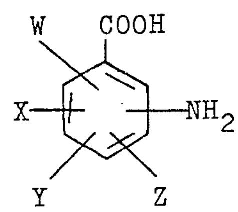

(I)

in welcher W, X, Y und Z zueinander gleiche oder voneinander verschiedene Bedeutungen haben können und jedes Wasserstoff, Fluor, Chlor, Brom, Amino, Nitro, Cyan, Carbamoyl, Sulfamoyl, Carboxy, Trifluormethyl, R′—, R′—O—, R′—O—CO—, R′—CO—, R′—NH—, R′R″N— oder R′—O—$SO_2$-Gruppen, in denen R′ und/oder R″ Alkyl mit 1 bis 10 C-Atomen sind, oder gegebenenfalls substituiertes Phenoxy, gegebenenfalls substituiertes Phenylcarbonylamino, gegebenenfalls substituiertes Phenoxamido oder gegebenenfalls substituiertes Phenylcarbamoyl, in welchen die Substituenten jeweils Alkyl und/oder Alkoxy mit je 1 bis 10 C-Atomen, Halogen, Carbamoyl und/oder gegebenenfalls halogensubstituiertes Phenoxy sind, bedeuten, oder in welcher W, X, Y, Z jeweils paarweise Ringatome eines ankondensierten carbocyclischen Ringsystems oder eines ankondensierten N-, O- und/oder S-haltigen heterocyclischen Ringsystems sind, die ihrerseits Oxofunktionen tragen können, durch Veresterung dieser Aminocarbonsäuren mit einem niederen Dialkylsulfat in einem aprotischen, mit Wasser mischbaren, nicht alkylierenden und nicht alkylierbaren organischen Lösemittel in Gegenwart einer basischen Verbindung, dadurch gekennzeichnet, daß man die Aminocarbonsäure der allgemeinen Formel (I) mit einer anorganischen Base in ein Carbonsäuresalz überführt und das Salz in einem wasserfreien Medium durch tropfenweise oder stetige Zugabe von Dialkylsulfat umsetzt oder daß man die Carbonsäure mit Dialkylsulfat vorlegt und bei nachfolgender tropfenweiser oder stetiger Zugabe von organischer Base in wasserfreiem Medium umsetzt.

Es werden insbesondere Aminocarbonsäuren der Formel I, in welcher W, X, Y und Z, die gleich oder verschieden sein können, Wasserstoff, Fluor, Chlor oder Brom, Carboxy, Amino, Nitro oder Trifluormethyl, ein Alkyl oder Alkoxy mit jeweils 1 bis 4 C-Atomen, Alkoxycarbonyl mit 2 bis 5 C-Atomen, ggf. substituiertes Phenylcarbamyl, worin die Substituenten Halogen, Carbamoyl und/oder ggf. chlorsubstituiertes Phenoxy sind, und/oder jeweils zei der Reste W, X, Y und Z eine ankondensierte Ureylen- oder (—NH—CO—CO—NH)-Gruppe sind, in der oben beschriebenen Weise umgesetzt.

Als Aminocarbonsäuren der Formel I kommen z. B in Frage :

Anthranilsäure, 3-, 4-, 5- oder 6-Chlor-anthranilsäure, 3-, 4- oder 5-Methylanthranilsäure, 3-, 4- oder 5-Nitro-anthranilsäure, 3-Amino-benzoesäure, 4-Amino-benzoesäure, 2-Amino-toluol-4-carbonsäure, 3-Amino-toluol-6-carbonsäure, 4-Chlor-3-amino-benzoesäure, 2-Chlor-4-amino-benzoesäure, 3-Chlor-4-amino-benzosäure, 5-Brom-2-amino-benzoesäure, 5-Fluor-2-amino-benzoesäure, 3,5-Dichlor-2-amino-benzoesäure, 3,6-Dichlor-5-aminobenzoesäure, 2,4-Dichlor-5-amino-benzoesäure, 3,4,5-Trichlor-2-amino-benzoesäure, 4-Trifluormethyl-2-amino-benzoesäure, 5-Chlor-2-methyl-4-amino-benzoesäure, 4-Nitro-3-amino-benzoesäure, 5-Nitro-3-amino-benzoesäure, 6-Nitro-3-amino-benzoesäure, 2-Nitro-4-amino-benzoesäure, 3-Nitro-4-amino-benzoesäure, 2,4-Diaminobenzoesäure, 3,4-Diaminobenzoesäure, 3,5-Diamino-benzoesäure, 3-Amino-4-methoxy-benzoesäure, 4-Amino-2,5-dimethoxy-benzoesäure, 2-Amino-terephthalsäure, 3-Amino-isophthalsäure, 4-Aminophthalsäure, 2-Amino-terephthalsäure-1-methylester, 2-Amino-terephthalsäure-4-methyl-ester, 2-Amino-4-[4-phenoxy-phenylcarbamoyl]-benzoesäure, 2-Amino-4-[4-(2,5-dichlor-phenoxy-)-phenylcarbamoyl]-benzoesäure, 2-Amino-4-[4-(2,5-dichlor-phenoxy-)-phenylcarbamoyl]-benzoesäure, 2-Amino-4-[4-carbamoylphenylcarbamoyl]-benzoesäure, 2-Amino-4-[3-carbamoyl-phenylcarbamoyl]-benzoesäure, 6-Amino-2-oxo-(1H)-benzimidazol-5-carbonsäure, 7-Amino-2,3-dioxo-(1H, 4H)-chinoxalin-6-carbonsäure, 2-Amino-4-[2,5-dichlor-phenylcarbamoyl]-benzoesäure.

Niedere Dialkylsulfate sind solche mit Alkylgruppen mit 1 bis 3 C-Atomen, z. B. Dimethylsulfat, Diethylsulfat oder Diisopropylsulfat.

Als aprotische mit Wasser vorzugsweise vollständig mischbare organische Lösemittel, die weder alkylierend wirken noch alkylierbar sind, können beispielsweise eingesetzt werden : Dimethylformamid, Dimethylsulfoxid, N-Methylpyrrolidon, Dimethylacetamid, Tetramethylharnstoff, Phosphorsäuretrisdimethylamid, Dimethylsulfon, Tetramethylensulfon, Aceton, Methylethylketon, Diethylketon, Acetonitril, Tetrahydrofuran, Dioxan, Diethylenglykoldimethylether, 1,2-Dimethoxyethan oder Acetophenon.

Die Selektivität der Esterbildung ist beim Einsatz von dipolar aprotischen, wassermischbaren Lösemitteln (z. B. N-Methylpyrrolidon, Dimethylformamid) am größten.

Als basische Verbindungen kommen beispielsweise in Betracht : anorganische Basen wie Kaliumhydroxid, Natriumhydroxid, Calciumoxid, Bariumoxid oder Salze wie Kaliumcarbonat, Kaliumhydrogencarbonat, Natriumcarbonat, Natriumhydrogencarbonat. Beim Einsatz von Kaliumverbindungen ist die Selektivität am größten.

Außerdem können genügend starke organische Basen wie z. B. Triethanolamin, Triethylamin, Dimethylamin oder Pyridin eingesetzt werden.

Es wird bei Temperaturen zwischen – 10 °C und 200 °C verestert, vorzugsweise zwischen 0 °C und 80 °C.

Beim Vorliegen der freien Säure werden wasserfreie anorganische Basen zur Salzbildung und als Protonenacceptoren eingesetzt, wobei das Salz vor der Veresterung gebildet und das hierbei entstehende Reaktionswasser vor der Veresterung abdestilliert bzw. gebunden wird.

Bezogen auf die eingesetzte Aminobenzoesäure bzw. deren Salz wird die zwei- bis dreißig-fache, vorzugsweise drei- bis fünfzehnfache Menge an Lösemittel benötigt. Die Salzbildung wird beim Einsatz anorganischer Basen oder Salze vor der Dialkylsulfatzugabe vorgenomen und verläuft bei Temperaturen zwischen 0 °C und 160 °C, vorzugsweise zwischen 60 °C und 130 °C, durch Zugabe der äquivalenten Menge Base. Die Reaktion verläuft nur dann selektiv, wenn das bei der Salzbildung entstehende Wasser durch Abdestillation (ggf. als Azeotrop) oder durch Zugabe von inerten wasserbindenden Mitteln, z. B. von Kupfer-(II)-sulfat, Calciumchlorid, Calciumsulfat, Natriumsulfat, Calciumoxid, Magnesiumoxid, Kieselgel oder ein Molekularsieb vor der Dialkylsulfatzugabe entfernt oder gebunden wird.

Das Dialkylsulfat wird mindestens in äquimolarer Menge zu den zu veresternden Carboxylgruppen eingesetzt, wobei ein geringer Überschuß von etwa 2-10 % vorteilhaft ist. Die Reaktionszeit läßt sich im wesentlichen an Hand der Temperatur steuern und liegt bei 15 Minuten bis 4 Stunden, normalerweise bei ca. 30 Minuten. Die Veresterung ist in der Regel unmittelbar nach dem Zutropfen des Dialkylsulfates abgeschlossen und verläuft sowohl in Lösung als auch in Suspension. Die pH-Werte der Reaktionsmischung, die sich erst nach Zugabe von Wasser zu einer Probe feststellen lassen, liegen während der Reaktion zwischen 5 und 12, vorzugsweise zwischen 7 und 11,5.

Beim Einsatz organischer Basen wird aus Gründen der Selektivität in umgekehrter Reinhenfolge verfahren, indem zunächst das Dialkylsulfat und danach die organische Base zur Reaktionslösung bzw. Suspension zugegeben wird.

Die Aufarbeitung der erhaltenen Ester geschieht dadurch, daß das Lösemittel vollständig oder teilweise abdestilliert und/oder mit Wasser bzw. mit einem geeigneten organischen Lösemittel der Ester ausgefällt wird. Vor der Destillation des Lösemittels kann vom evtl. ausgefällten Salz des Monoalkylsulfates abgesaugt werden. Flüssige Ester werden destillativ vom Lösemittel getrennt, wasserdampfflüchtige Lösemittel werden mit Hilfe der Wasserdampfdestillation vom Reaktionsprodukt (Ester) abgeschieden.

Das beschriebene Verfahren zeichnet sich durch hohe Ausbeuten und Reinheit der erhaltenen Ester aus.

## Beispiel 1

51,5 g 2-Amino-4-chlor-benzoesäure werden in die vorgelegte Suspension von 256 g N,N-Dimethylformamid und 30,6 g Kaliumcarbonat (gemahlen) eingetragen und 30 Min. auf 125 °C erhitzt. Danach wird über eine Kolonne das Reaktionswasser und etwas Dimethylformamid abdestilliert, auf 10 °C gekühlt und in 60 Minuten 39,7 g Dimethylsulfat bei 10 bis 12 °C zugetropft. Es wird etwa eine Stunde bei 10 °C und danach eine Stunde bei Raumtemperatur nachgerührt, von ausgefallenen Salzen

abgesaugt, auf 1,7 kg Eiswasser (angesäuert mit 0,3 ml Salzsäure) gegeben, abgesaugt, portionsweise mit etwa 400 g Eiswasser gewaschen und bei 50 °C im Vakuum getrocknet.

Ausbeute : 53,1 g 2-Amino-4-chlor-benzoesäuremethylester (95,4 % d. Th.) ; Fp. 68,5 °C.
Gaschromatogramm (GC) : Frühel. Komp. < 0,1 %
2-Methylamino-4-chlor-benzoesäuremethylester : 1,4 %
Spätel. Komp. 0,2 %.

Beispiel 2

In eine vorgelegte Suspension von 263 g Dimethylformamid und 5,4 g Kaliumcarbonat (gemahlen) werden 52,6 g Kaliumsalz der p-Aminobenzoesäure (gemahlen) eingetragen und 15 Min. unter Feuchtigkeitsausschluß bei 120 °C gerührt. Danach werden bei 70 °C 45,5 g Dimethylsulfat in etwa 30 Minuten zugetropft und 60 Minuten bei 70 °C nachgerührt. Es wird von ausgefallenen Salzen (im wesentlichen Kalium-Monomethylsulfat) abgesaugt und unter Wasserstrahlvakuum das Lösemittel abdestilliert. Der Ansatz wird in der Hitze (etwa 70 °C) mit 39,5 g (50 ml) Methanol versetzt und bei Raumtemperatur unter Rühren auf etwa 500 g Eiswasser gegeben, das mit etwa 0,5 g Salzsäure (31 %ig) angesäuert ist. Es wird abgesaugt, mit etwa 150 g Eiswasser neutral and lösemittelfrei gewaschen und bei 50 °C im Vakuum getrocknet.

Ausbeute : 43,9 g p-Aminobenzoesäuremethylester (97 % d. Th.) ; Fp. 112 °C.
GC : frühel. Komp. < 0,1 %
spätel. Komp. 2,4 %
p-Aminobenzoesäure : 1,5 % (durch Titration bestimmt).

Beispiel 3

In eine vorgelegten Suspension von 30 g N,N-Dimethylacetamid und 1,7 g Kaliumcarbonat werden 9,7 g 2-Aminoterephthalsäure-1-methylester eingetragen und 30 Minuten auf 100 °C erhitzt. Es wird auf 70 °C abgekühlt, 1,7 g Kaliumcarbonat und 3,5 g wasserfreies Natriumsulfat zugegeben und in etwa 30 Minuten 6,6 g Dimethylsulfat zugetropft, wobei während des Zutropfens weitere 1,7 g Kaliumcarbonat zugesetzt werden. Es wird 10 Minuten bei 70 °C nachgerührt, auf Raumtemperatur gekühlt, 60 ml Eiswasser zugetropft, abgesaugt, mit Eiswasser neutral gewaschen und bei 50 °C im Vakuum getrocknet.

Ausbeute : 9,8 g 2-Amino-terephthalsäure-dimethylester (94 % d. Th.) ; Fp : 133-134 °C.

Beispiel 4

In eine vorgelegte Suspension von 204 g N,N-Dimethylacetamid und 13,6 g Kalium-carbonat (gemahlen) werden 18,1 g 5-Amino-isophthalsäure eingetragen und 30 Minuten bei 50 °C gerührt. Es wird auf 20 °C abgekühlt, mit 6,8 g Kaliumcarbonat (gemahlen) und 7,1 g wasserfreiem Natriumsulfat versetzt, bei 20 °C in etwa 30 Minuten 27,1 g Dimethylsufat zugetropft und 3 Stunden bei 20 °C nachgerührt. Nach vollständiger Umsetzung wird der Ansatz auf 800 g Eiswasser gegeben, das mit 2,5 g Salzsäure (31 %ig) angesäuert ist. Es wird mit Eiswasser neutral gewaschen und bei 50 °C im Vakuum getrocknet.

Ausbeute : 17,8 g 5-Amino-isophthalsäuredimethylester (85 % d. Th.) ; Fp. : 176 °C.

Beispiel 5

86 g N,N-Dimethylacetamid und 17,1 g Natriumcarbonat (gemahlen) werden vorgelegt, 18,2 g 2-Amino-4-nitrobenzoesäure eingetragen und etwa 30 Minuten auf 120 °C erhitzt. Danach werden etwa 20 g Lösemittel-Wasser-Gemisch abdestilliert und das fehlende Lösemittel im Reaktionsgefäß wieder ersetzt. Es wird auf 30 °C gekühlt, in ca. 30 Minuten 20,1 g Diethylsulfat zugetropft und 2 Stunden bei 30 °C nachgerührt. Nach vollständiger Umsetzung wird die Reaktionslösung langsam auf 500 g Eiswasser gegeben, das mit 4,5 g Salzsäure (31 %ig) angesäuert ist. Anschließend wird abgesaugt, neutral und salzfrei gewaschen und bei 60 °C im Vakuum getrocknet.

Ausbeute : 20,2 g 2-Amino-4-nitro-benzoesäureethylester (97,0 % d. Th.) ; Fp. : 100 °C.

Vergleichbare Ergebnisse werden mit N-Methylpyrrolidon anstelle von N,N-Dimethylacetamid erhalten.

In der folgenden Tabelle sind noch weitere Beispiele für Ester aufgeführt, die entsprechend Beispiel 1 bis 5 erhalten werden.

(Siehe Tabelle Seite 5 f.)

| | Fp. [°C] |
|---|---|
| Anthranilsäure-methylester | 24-25 |
| -ethylester | 13,0 |
| 5-Brom-anthranilsäure-methylester | 74 |
| -ethylester | 187 |
| 4-Chlor-anthranilsäure-ethylester | 41 |
| 5-Chlor-anthranilsäure-methylester | 76 |
| 6-Chlor-anthranilsäure-methylester | Kp.: 156-159°C/13 mbar |
| 3-Methyl-anthranilsäure-methylester | Kp.: 153°C/30 mbar |
| 4-Methyl-anthranilsäure-ethylester | 44 |
| 3-Nitro-anthranilsäure-ethylester | 109 |
| 4-Nitro-anthranilsäure-methylester | 157 |
| 5-Nitro-anthranilsäure-methylester | 168 |
| " -ethylester | 145 |
| 4-Trifluormethyl-anthranilsäure-methylester | |
| 3-Amino-benzoesäure-methylester | 36-38 |
| " -ethylester | 84-85 |
| 3-Amino-4-chlor-benzoesäure-methylester | 88-89 |
| 3-Amino-4-fluor-benzoesäure-methylester | 72-73 |
| 3-Amino-4-methoxy-benzoesäure-methylester | 85-86 |
| 3-Amino-4-nitro-benzoesäure-ethylester | 139 |
| 3-Amino -5-nitro-benzoesäure-methylester | 158-160 |
| " -ethylester | 155 |
| 3-Amino-6-nitro-benzoesäure-ethylester | 107,5 |
| 4-Amino-benzoesäure-ethylester | 91-92 |
| 4-Amino-2-chlor-benzoesäure-methylester | 111-112 |
| 4-Amino-3-chlor-benzoesäure-methylester | 113-114 |
| 4-Amino-2-nitro-benzoesäure-methylester | 157-159,5 |
| " -ethylester | 130 |
| 4-Amino-3-nitro-benzoesäure-methylester | 199,5-200 |
| " -ethylester | 136 |

(Fortsetzung)

| | Fp. [°C] |
|---|---|
| 4-Amino-3,5-dimethoxy-benzoesäure-ethylester | 45 |
| 5-Amino-2,4-dichlor-benzoesäure-methylester | 58-60 |
| 2,5-Diamino-benzoesäure-ethylester | 50,5-51 |
| 3,4-Diamino-benzoesäure-methylester | 108-109 |
| " -ethylester | 112-113 |
| 3,5-Diamino-benzoesäure-methylester | 84 |
| 5-Chlor-3-nitro-2-amino-benzoesäure-methylester | 110-112 |
| 3-Chlor-anthranilsäure-methylester | 37,5-38 |
| 3-Amino-2,5-dichlorbenzoesäure-methylester | 57 |
| -ethylester | 64 |
| 2-Amino-4-[4-phenoxy-phenylcarbamoyl]-benzoesäure-methylester | 172-175 |
| 2-Amino-4-[4-(2,5-dichlor-phenoxy)-phenylcarbamoyl]-benzoesäure-methylester | 217 |
| 2-Amino-4-[4-carbamoyl-phenylcarbamoyl]-benzoesäure-methylester | 308 |
| 2-Amino-4-[3-carbamoyl-phenylcarbamoyl]-benzoesäure-methylester | 230-232 |
| 6-Amino-2-oxo-(1H)-benzimidazol-5-carbonsäure-methylester | 295-297 |
| 7-Amino-2,3-dioxo-(1H, 4H)-chinoxalin-6-carbonsäure-methylester | 350 |
| 2-Amino-4-[2,5-dichlor-phenylcarbamoyl]-benzoesäure-methylester | 207-208 |

**Patentansprüche**

1. Verfahren zur Herstellung von niederen aromatischen Aminocarbonsäurealkylestern von Aminocarbonsäuren der allgemeinen Formel (I)

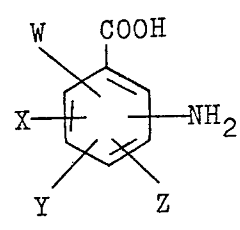

(I)

in welcher W, X, Y und Z zueinander gleiche oder voneinander verschiedene Bedeutungen haben können und jedes Wasserstoff, Fluor, Chlor, Brom, Amino, Nitro, Cyan, Carbamoyl, Sulfamoyl, Carboxy, Trifluormethyl, R'—, R'—O—, R'—O—CO—, R'—CO—, R'—NH—, R'R"N— oder R'—O—$SO_2$-Gruppen, in denen R' und/oder R" Alkyl mit 1 bis 10 C-Atomen sind, oder gegebenenfalls substituiertes Phenoxy, gegebenenfalls substituiertes Phenylcarbonylamino, gegebenenfalls substituiertes Phenoxamido oder gegebenenfalls substituiertes Phenylcarbamoyl, in welchen die Substituenten jeweils Alkyl und/oder Alkoxy mit je 1 bis 10 C-Atomen, Halogen, Carbamoyl und/oder gegebenenfalls halogensubstituiertes Phenoxy sind, bedeuten, oder in welcher W, X, Y, Z jeweils paarweise Ringatome eines ankondensierten carbocyclischen Ringsystems oder eines ankondensierten N—, O— und/oder S-haltigen heterocyclischen Ringsystems sind, die ihrerseits Oxofunktionen tragen können, durch Veresterung dieser Aminocarbonsäuren mit einem niederen Dialkylsulfat in einem aprotischen, mit Wasser mischbaren, nicht alkylierenden und nicht alkylierbaren organischen Lösemittel in Gegenwart einer basischen Verbindung, dadurch gekennzeichnet, daß man die Aminocarbonsäure der allgemeinen Formel (I) mit einer anorganischen Base in ein Carbonsäuresalz überführt und das Salz in einem wasserfreien Medium durch tropfenweise oder stetige Zugabe von Dialkylsulfat umsetzt oder daß man die Carbonsäure mit Dialkylsulfat vorlegt und bei nachfolgender tropfenweiser oder stetiger Zugabe von organischer Base in wasserfreiem Medium umsetzt.

2. Verfahren nach Anspruch 1, dadurch gekennzeichnet, daß man Aminocarbonsäuren der allgemeinen Formel I, in welcher W, X, Y und Z, die gleich oder verschieden sein können, Wasserstoff, Fluor, Chlor oder Brom, Carboxy, Amino, Nitro oder Trifluormethyl, Alkyl oder Alkoxy mit jeweils 1 bis 4 C-Atomen, Alkoxycarbonyl mit 2 bis 5 C-Atomen, gegebenenfalls substituiertes Phenylcarbamoyl, worin die Substituenten Halogen, Carbamoyl und/oder gegebenenfalls Chlor- substituiertes Phenoxy sind, und/oder jeweils zwei der Reste W, X, Y und Z eine ankondensierte Ureylen- oder (—NH—CO—CO—NH)-Gruppe sind, umsetzt.

3. Verfahren nach Anspruch 1 und 2, dadurch gekennzeichnet, daß man Aminosäuren der allgemeinen Formel I, in welcher W, X, Y und Z, die gleich oder verschieden sein können, Chlor, Nitro oder Trifluormethyl oder gegebenenfalls mit Chlor substituiertes Phenylcarbamoyl sind, umsetzt.

4. Verfahren nach Anspruch 1 bis 3, dadurch gekennzeichnet, daß man Salze anorganischer Basen umsetzt.

5. Verfahren nach Anspruch 1 bis 4, dadurch gekennzeichnet, daß man die Kalium- oder Natriumsalze umsetzt.

6. Verfahren nach Anspruch 1 bis 5, dadurch gekennzeichnet, daß man die Salze der Aminocarbonsäure in situ durch Umsetzung der Aminocarbonsäuren mit basischen Verbindungen herstellt.

7. Verfahren nach Anspruch 6, dadurch gekennzeichnet, daß man das Reaktionswasser aus der Salzbildung vor der Veresterung entfernt.

8. Verfahren nach Anspruch 7, dadurch gekennzeichnet, daß man das Reaktionswasser destillativ als Komponente eines azeotropen Gemisches oder durch Zugabe eines wasserbindenden Mittels entfernt.

9. Verfahren nach Anspruch 1 bis 5, dadurch gekennzeichnet, daß die Umsetzung bei − 10 bis 200 °C erfolgt.

**Claims**

1. A process for the manufacture of an aromatic aminocarboxylic acid lower alkyl ester from an aminocarboxylic acid of the general formula (I)

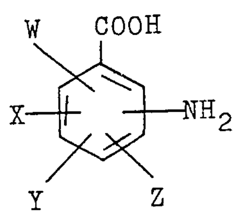

(I)

in which W, X, Y and Z, being identical or different, each denotes hydrogen, fluoro, chloro, bromo, amino, nitro, cyano, carbamoyl, sulfamoyl, carboxy, trifluoromethyl, R'—, R'—O—, R'—O—CO, R'—CO—, R'—NH—, R'R"N— or R'—O—$SO_2$—, R' and/or R" being alkyl having from 1 to 10 carbon atoms, optionally substituted phenoxy, optionally substituted phenylazo, optionally substituted phenylcarbonylamino, optionally substituted phenoxamide or optionally substituted phenylcarbamoyl, the substituents being in each case alkyl and/or alkoxy each having from 1 to 10 carbon atoms, halogeno, carbamoyl and/or phenoxy optionally substituted by halogen, or in which two of W, X, Y and Z are ring atoms of a fused carboxyclic ring system of a fused N—, O— and/or S-containing heterocyclic ring system, which for their part may carry oxo functions, wherein said aminocarboxylic acid is esterified with a lower dialkyl sulfate in an aprotic, water-miscible, non-alkylating and non-alkylatable organic solvent in the presence of a basic compound, characterized by converting the aminocarboxylic acid into its

carboxylic acid salt by means of an inorganic base and reacting the salt obtained in anhydrous medium through dropwise or continual addition of the dialkyl sulfate, or by initially introducing the aminocarboxylic acid together with the dialkyl sulfate and then reacting the mixture through dropwise or continual addition of an organic base in an anhydrous reaction medium.

2. The process as claimed in Claim 1, characterized by reacting an aminocarboxylic acid of the general formula I, in which W, X, Y and Z, being identical or different, each are hydrogen, fluoro, chloro or bromo, carboxy, amino, nitro or trifluoromethyl, alkyl or alkoxy having each from 1 to 4 carbon atoms, alkoxycarbonyl having from 2 to 5 carbon atoms, optionally substituted phenylcarbamoyl, the substituents being halogeno, carbamoyl and/or two of the radicals W, X, Y and Z each are a fused ureylene or (—NH—CO—CO—NH—) group.

3. The process as claimed in Claim 1 and 2, characterized by reacting an amino acid of the formula I, in which W, X, Y and Z, being identical or different, each are chloro, nitro, trifluoromethyl or phenylcarbamoyl which is optionally substituted by chloro.

4. The process as claimed in Claim 1 to 3, characterized by reacting a salt of an inorganic base.

5. The process as claimed in Claim 1 to 4, characterized by reacting a potassium or sodium salt.

6. The process as claimed in Claim 1 to 5, characterized by preparing the salt of the aminocarboxylic acid in situ by reaction of the aminocarboxylic acid with a basic compound.

7. The process as claimed in Claim 6, characterized by eliminating the water of reaction from the salt formed before esterification.

8. The process as claimed in Claim 7, characterized by eliminating the water of reaction by distillation as component of an azeotropic mixture of by addition of a water-binding agent.

9. The process as claimed in Claim 1 to 5, characterized by carrying out the reaction at − 10° to 200 °C.

**Revendications**

1. Procédé de préparation d'esters alkyliques inférieurs d'acides amino-carboxyliques aromatiques, ces acides répondant à la formule (I) :

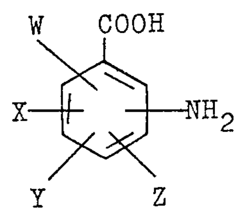

(I)

dans laquelle W, X, Y et Z représentent chacun, indépendamment les uns des autres, un atome d'hydrogène, de fluor, de chlore ou de brome ou un radical amino, nitro, cyano, carbamoyle, sulfamoyle, carboxy, trifluorométhyle, R'—, R'—O—, R'—O—CO, R'—CO—, R'—NH—, R'R"N— ou R'—O—$SO_2$— (le symbole R' et/ou le symbole R", dans ces radicaux, représentant un alkyle en $C_1$-$C_{10}$), un phénoxy éventuellement substitué, un phénylcarbonylamino éventuellement substitué, un phénoxamido éventuellement substitué ou un phénylcarbamoyle éventuellement substitué (comme substituants, sur chacun de ces radicaux, il peut y avoir un alkyle en $C_1$-$C_{10}$ et/ou un alcoxy en $C_1$-$C_{10}$, un halogène, un carbamoyle et/ou un phénoxy éventuellement halogéné), ou dans laquelle W, X, Y et Z sont assemblés par couple et sont alors des atomes appartenant à un système carbocyclique condensé ou à un système hétérocyclique condensé contenant un atome d'azote, d'oxygène et/ou de soufre, ces cycles pouvant eux-mêmes porter des fonctions oxo, par estérification de ces acides aminocarboxyliques avec un sulfate de dialkyle inférieur, dans un solvant organique aprotique, miscible à l'eau, non alkylant et non alkylable, en présence d'un composé basique, procédé caractérisé en ce qu'on transforme l'acide aminocarboxylique de formule générale (I), au moyen d'une base minérale, en un sel et on fait réagir celui-ci, dans un milieu anhydre, en y ajoutant goutte à goutte ou constamment le sulfate de dialkyle, ou encore on place dès le départ dans le récipient l'acide carboxylique et le sulfate de dialkyle et on les fait réagir en milieu anhydre en y ajoutant ensuite, goutte à goutte ou constamment, une base organique.

2. Procédé selon la revendication 1 caractérisé en ce qu'on fait réagir des acides aminocarboxyliques de formule générale I dans lesquels W, X, Y et Z représentent chacun, indépendamment les uns des autres, un atome d'hydrogène, de fluor, de chlore ou de brome, un radical carboxy, amino, nitro ou trifluorométhyle, un alkyle ou un alcoxy contenant chacun de 1 à 4 atomes de carbone, un alcoxycarbonyle contenant de 2 à 5 atomes de carbone, ou un phénylcarbamoyle éventuellement substitué (comme substituants celui-ci pourra porter un halogène, un carbamoyle et/ou un phénoxy éventuellement chloré), et/ou un ou deux des couples formés à partir de W, X, Y et Z est (ou sont) un radical uréylène ou —NH—CO—CO—NH— condensé.

3. Procédé selon l'une des revendications 1 et 2, caractérisé en ce qu'on fait réagir des acides aminés

de formule générale I dans lesquels W, X, Y et Z représentent chacun, indépendamment les uns des autres, un atome de chlore, un radical nitro ou trifluorométhyle ou un radical phénylcarbamoyle éventuellement chloré.

4. Procédé selon l'une quelconque des revendications 1 à 3, caractérisé en ce qu'on fait réagir des sels de bases minérales.

5. Procédé selon l'une quelconque des revendications 1 à 4, caractérisé en ce qu'on fait réagir les sels potassiques ou sodiques.

6. Procédé selon l'une quelconque des revendications 1 à 5, caractérisé en ce qu'on prépare les sels de l'acide aminocarboxylique in situ par réaction des acides aminocarboxyliques avec des composés basiques.

7. Procédé selon la revendication 6, caractérisé en ce qu'on élimine, avant l'estérification, l'eau provenant de la réaction de salification.

8. Procédé selon la revendication 7, caractérisé en ce que l'eau engendrée par la réaction est éliminée par distillation sous la forme d'une composante d'un mélange aséotrope ou par addition d'un accepteur d'eau.

9. Procédé selon l'une quelconque des revendications 1 à 5, caractérisé en ce que la réaction est effectuée à une température de −10 à 200 °C.